# EUROPÄISCHE PATENTANMELDUNG

(11) **EP 1 733 728 A2**
(43) Veröffentlichungstag der Anmeldung: **20.12.2006**
(21) Anmeldenummer: 06017257.4
(22) Anmeldetag: 17.07.2002
(51) Int. Cl.: A61K 31/53, A61P 17/06, A61P 15/08, A61P 35/00, A61P 3/10, A61P 27/06, A61P 1/00, A61P 11/12, A61P 15/06, A61P 13/10, A61P 13/08, A61P 17/14, A61P 25/16

(54) **Verwendung von 2-Alkoxphenyl-substituierten Imidazotriazinonen als Phosphodiesterase-Hemmer**

(30) Priorität: 23.07.2001 DE 10135815
(62) Teilanmeldung aus: 02748858.4
(71) Anmelder: Bayer HealthCare AG, 51368 Leverkusen (DE)
(72) Erfinder: Niewöhner, Ulrich, verstorben (DE); Bischoff, Erwin, 42115 Wuppertal (DE); Haning, Helmut, 42327 Wuppertal (DE); Rahbar, Afssaneh, 50226 Frechen (DE); Bandel, Tiemo-Jörg, 46145 Oberhausen (DE); Barth, Wolfgang, 42349 Wuppertal (DE)

(57) **Zusammenfassung**

Die vorliegende Erfindung betrifft die Verwendung von bekannten 2-Phenylsubstituierten Imidazotriazinonen mit kurzen, unverzweigten Alkylresten in der 9-Position und cGMP-PDE-inhibitorischen Eigenschaften zur Herstellung von Arzneimitteln zur Behandlung von Psoriasis, weiblicher Infertilität, Krebs, Diabetes, Augenerkrankungen wie Glaukom, Störungen der Magenbeweglichkeit, zystischer Fibrose, vorzeitigen Wehen, Blasenerkrankungen, Prostatahyperplasie, nitratinduzierte Toleranz, Präeklampsie, Alopecia, der Parkinson-Krankheit, Schmerz, Tinnitus oder dem renalen Syndrom.

## Beschreibung

Die vorliegende Erfindung betrifft die Verwendung von 2-Alkoxyphenyl-substituierten Imidazotriazinone zur Herstellung von Arzneimitteln zur Behandlung von Herzinsuffizienz, Psoriasis, weiblicher Infertilität, Krebs, Diabetes, Augenerkrankungen wie Glaukom, Störungen der Magenbeweglichkeit, zystischer Fibrose, vorzeitigen Wehen, pulmonarem Bluthochdruck, Blasenerkrankungen, Prostatahyperplasie, nitrat-induzierte Toleranz, Präeklampsie, Alopecia, der Parkinson-Krankheit, Schmerz, Tinnitus oder dem renalen Syndrom.

In der Offenlegungsschrift DE 28 11 780 sind Imidazotriazinone als Bronchodilatoren mit spasmolytischer Aktivität und Hemmaktivität gegen cyclisches Adenosin-monophosphat metabolisierende Phosphodiesterasen (cAMP-PDE's, Nomenklatur nach Beavo: PDE-III und PDE-IV) beschrieben. Eine Hemmwirkung gegen cyclisches Guanosin-monophosphat metabolisierende Phosphodiesterasen (cGMP-PDE's, Nomenklatur nach Beavo und Reifsnyder (Trends in Pharmacol. Sci. 11, 150-155, 1990) PDE-I, PDE-II und PDE-V) ist nicht beschrieben. Es werden keine Verbindungen beansprucht, die eine Sulfonamidgruppe im Arylrest in der 2-Position enthalten. Weiterhin werden Imidazotriazinone in FR 22 13 058, CH 59 46 71, DE 22 55 172, DE 23 64 076 und EP 000 9384 beschrieben, die in der 2-Position keinen substituierten Arylrest besitzen, und ebenfalls als Bronchodilatatoren mit cAMP-PDE inhibitorischer Wirkung beschrieben werden.

In WO 94/28902 werden Pyrazolopyrimidinone beschrieben, die sich für die Behandlung von Impotenz eignen.

In der WO 99/24433 und der WO 99/67244 sind Imidazotriazinone beschrieben, die sich für die Behandlung von Impotenz eignen

Zur Zeit sind in der Literatur 11 Phosphodiesterasen mit unterschiedlicher Spezifität gegenüber den cyclischen Nukleotiden cAMP und cGMP beschrieben (Vgl. Fawcett et al., Proc. Nat. Acad. Sci. 97(7), 3072-3077 (2000). Cyclisches Guanosin 3',5'-monophosphat metabolisierende Phosphodiesterasen (cGMP-PDE's) sind die PDE-1, 2, 5, 6, 9, 10, 11. Die erfindungsgemäßen Verbindungen sind potente Inhibitoren der Phosphodiesterase 5. Die differenzierte Expression der Phosphodiesterasen in verschiedenen Zellen, Geweben und Organen, ebenso wie die differenzierte subzelluläre Lokalisation dieser Enzyme, ermöglichen in Verbindung mit den erfindungsgemäßen selektiven Inhibitoren eine selektive Erhöhung der cGMP-Konzentration in spezifischen Zellen, Geweben und Organen und ermöglichen dadurch die Adressierung von verschiedenen von cGMP regulierten Vorgängen. Dies ist besonders zu erwarten wenn unter bestimmten physiologischen Bedingungen die Synthese von cGMP gesteigert ist. Zum Beispiel wird während sexueller Stimulation auf neuronalem Wege Stickstoffmonoxid in den Gefäßen des Corpus Cavernosum freigesetzt und damit die Synthese von cGMP gesteigert. Dies führt zu einer starken Erweiterung der Gefäße, die den Corpus Cavernosum mit Blut versorgen, und damit zur Erektion. Daher sollten Inhibitoren cGMP metabolisierender PDEs besonders für die Behandlung der erektilen Dysfunktion geeignet sein.

Ein Anstieg der cGMP-Konzentration kann zu heilsamen, antiaggregatorischen, antithrombotischen, antiproliferativen, antivasospastischen, vasodilatierenden, natriuretischen und diuretischen Effekten führen und kann die Erregungsleitung im zentralen Nervensystem und damit die Gedächtnisleistung beeinflussen. Es kann die Kurz- oder Langzeitmodulation der vaskulären und kardialen Inotropie, den Herzrhythmus und die kardiale Erregungsleitung beeinflussen (J.C. Stoclet, T. Keravis, N. Komas and C. Lugnier, Exp. Opin. Invest. Drugs (1995), 4 (11), 1081-1100).

Die vorliegende Erfindung betrifft die Verwendung von Verbindungen der allgemeinen Formel (I) 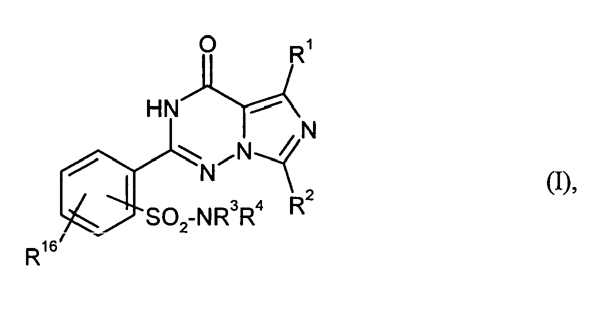 in welcher
- R¹: für Methyl oder Ethyl steht,
- R²: für Ethyl oder Propyl steht,
- R³ und R⁴: gleich oder verschieden sind und für eine geradkettige oder verzweigte Alkylkette mit bis zu 5 Kohlenstoffatomen stehen, die gegebenenfalls bis zu zweifach gleich oder verschieden durch Hydroxy oder Methoxy substituiert ist,
oder
- R³ und R⁴: gemeinsam mit dem Stickstoffatom einen Piperidinyl-, Morpholinyl-, Thiomorpholinylring oder einen Rest der Formel 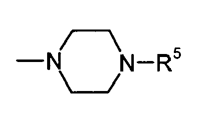 bilden, worin
R⁵ Wasserstoff, Formyl, Acyl oder Alkoxycarbonyl mit jeweils bis zu 3 Kohlenstoffatomen bedeutet, oder geradkettiges oder verzweigtes Alkyl mit bis zu 3 Kohlenstoffatomen bedeutet, das gegebenenfalls ein- bis zweifach, gleich oder verschieden durch Hydroxy, Carboxyl, geradkettiges oder verzweigtes Alkoxy oder Alkoxycarbonyl mit jeweils bis zu 3 Kohlenstoffatomen oder durch Gruppen der Formeln -(D)ₐ₋NR⁶R⁷ oder -P(O)(OR⁸)(OR⁹) substituiert ist,
worin
a eine Zahl 0 oder 1 bedeutet,
D eine Gruppe der Formel -CO bedeutet,
R⁶ und R⁷ gleich oder verschieden sind und Wasserstoff oder Methyl bedeuten,
R⁸ und R⁹ gleich oder verschieden sind und Wasserstoff, Methyl oder Ethyl bedeuten, oder
R⁵ Cyclopentyl bedeutet,
und die unter R³ und R⁴ aufgeführten, gemeinsam mit dem Stickstoffatom gebildeten Heterocyclen, gegebenenfalls ein- bis zweifach, gleich oder verschieden, gegebenenfalls auch geminal, durch Hydroxy, Formyl, Carboxyl, Acyl oder Alkoxycarbonyl mit bis jeweils zu 3 Kohlenstoffatomen oder Gruppen der Formeln -P(O)(OR¹⁰)(OR¹¹) oder -(CO)_{b}NR¹²R¹³ substituiert sind,
worin
- R¹⁰ und R¹¹: gleich oder verschieden sind und Wasserstoff, Methyl oder Ethyl bedeuten,
- b: eine Zahl 0 oder 1 bedeutet,
und
- R¹² und R¹³: gleich oder verschieden sind und Wasserstoff oder Methyl bedeuten
und/oder die unter R³ und R⁴ aufgeführten, gemeinsam mit dem Stickstoffatom gebildeten Heterocyclen, gegebenenfalls durch geradkettiges oder verzweigtes Alkyl mit bis zu 3 Kohlenstoffatomen substituiert sind, das gegebenenfalls ein- bis zweifach, gleich oder verschieden durch Hydroxy, Carboxyl oder durch einen Rest der Formel P(O)OR¹⁴OR¹⁵ substituiert ist,
worin
- R¹⁴ und R¹⁵: gleich oder verschieden sind und Wasserstoff, Methyl oder Ethyl bedeuten,
und/oder die unter R³ und R⁴ aufgeführten, gemeinsam mit dem Stickstoffatom gebildeten Heterocyclen, gegebenenfalls durch über N-verknüpftes Piperidinyl oder Pyrrolidinyl substituiert sind,
und
- R¹⁶: für Ethoxy oder Propoxy steht,
und deren Salze, Hydrate und/oder Solvate, zur Herstellung von Arzneimitteln zur Behandlung von Herzinsuffizienz, Psoriasis, weiblicher Infertilität, Krebs, Diabetes, Augenerkrankungen wie Glaukom, Störungen der Magenbeweglichkeit, zystischer Fibrose, vorzeitigen Wehen, pulmonarem Bluthochdruck, Blasenerkrankungen, Prostatahyperplasie, nitrat-induzierte Toleranz, Präeklampsie, Alopecia, der Parkinson-Krankheit, Schmerz, Tinnitus oder dem renalen Syndrom.

Besonders bevorzugt ist gemäß der vorliegenden Erfindung die Verwendung folgender Verbindungen:

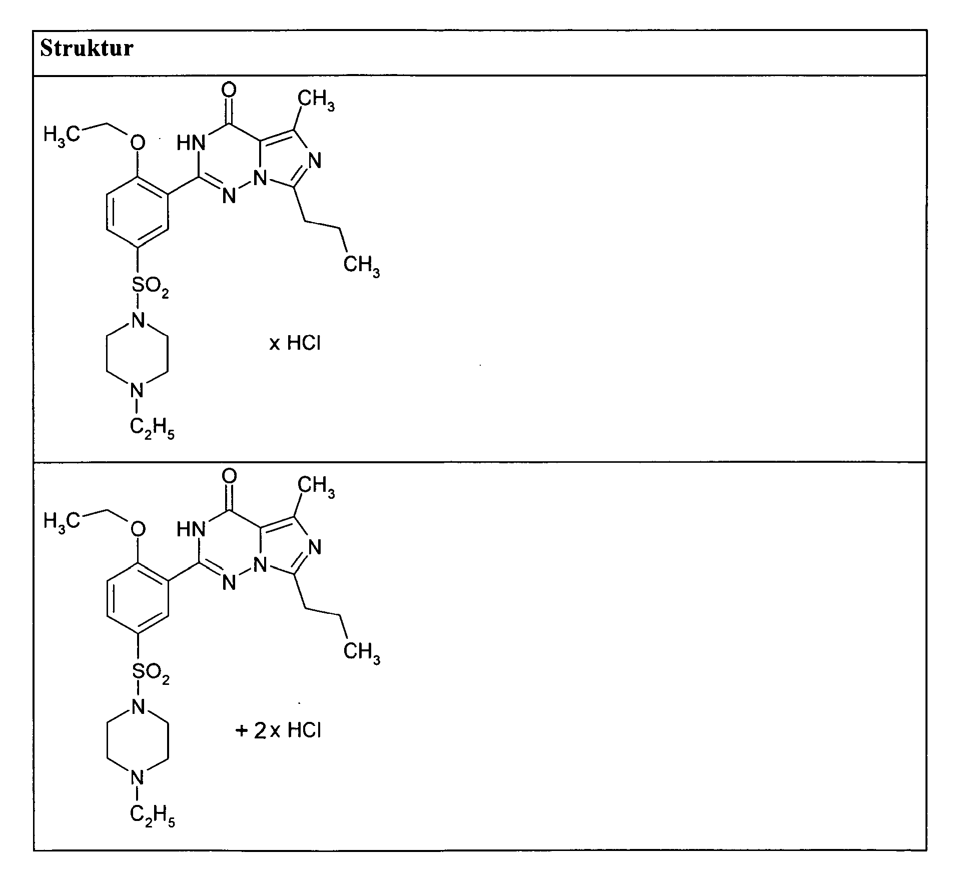

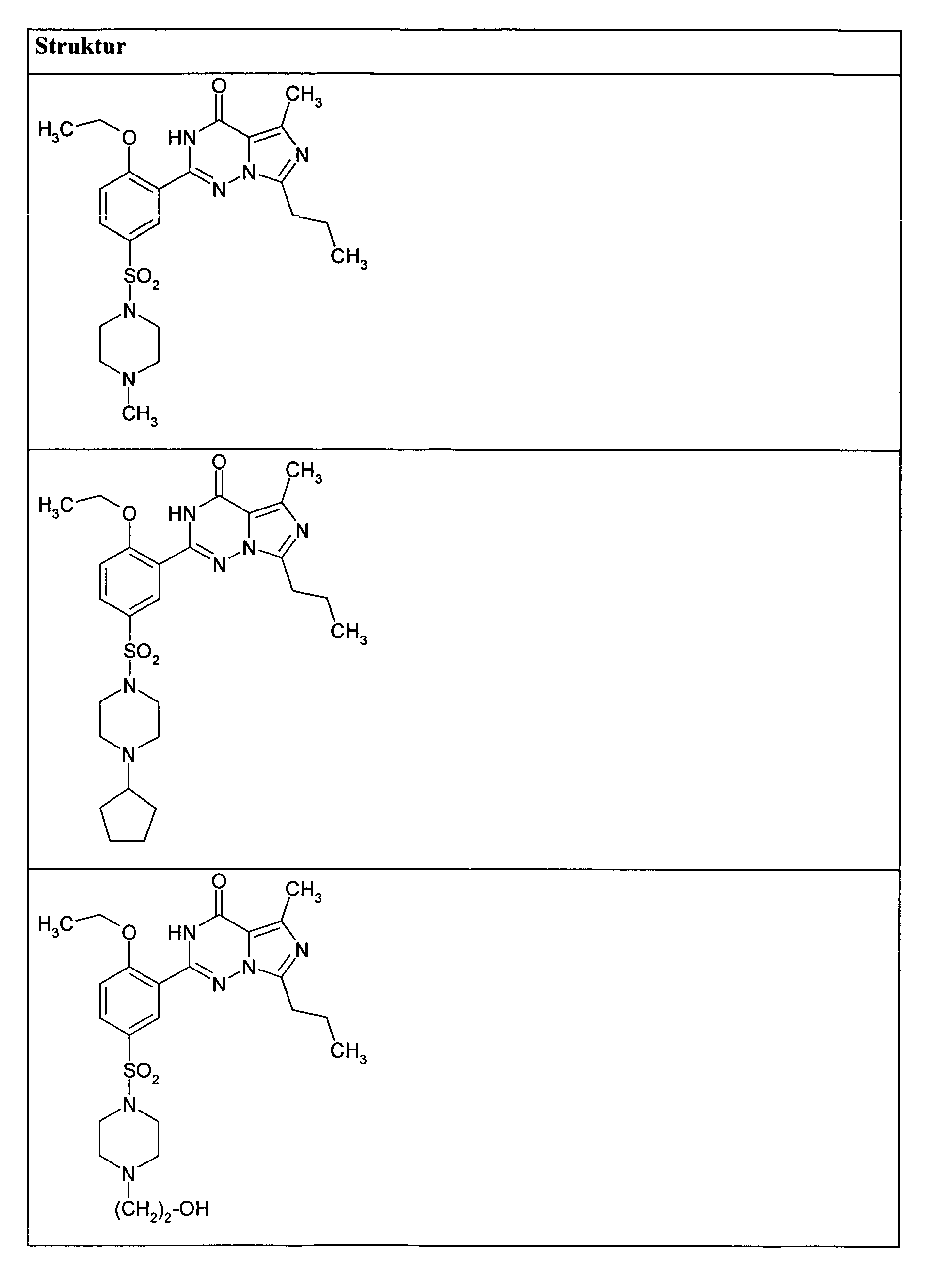

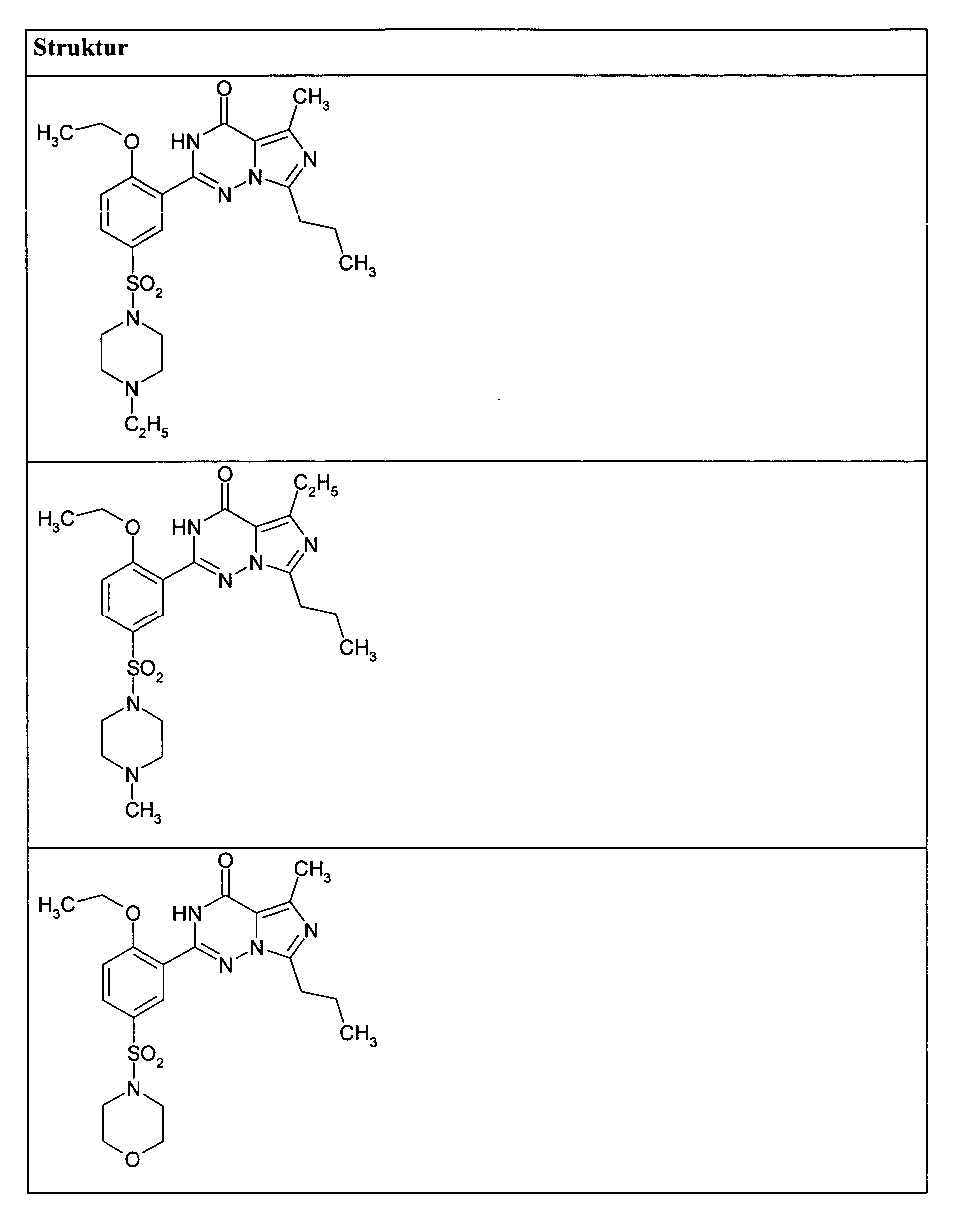

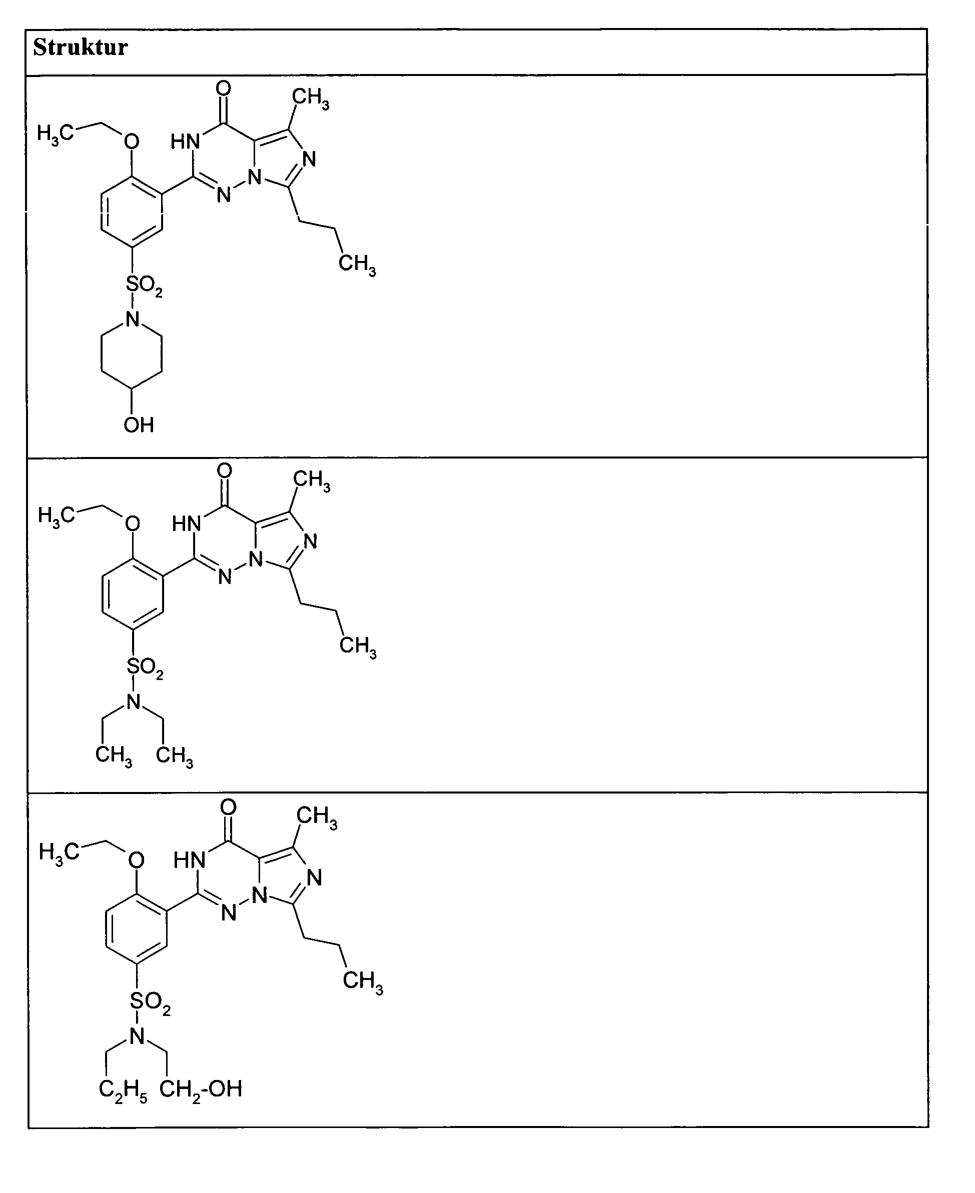

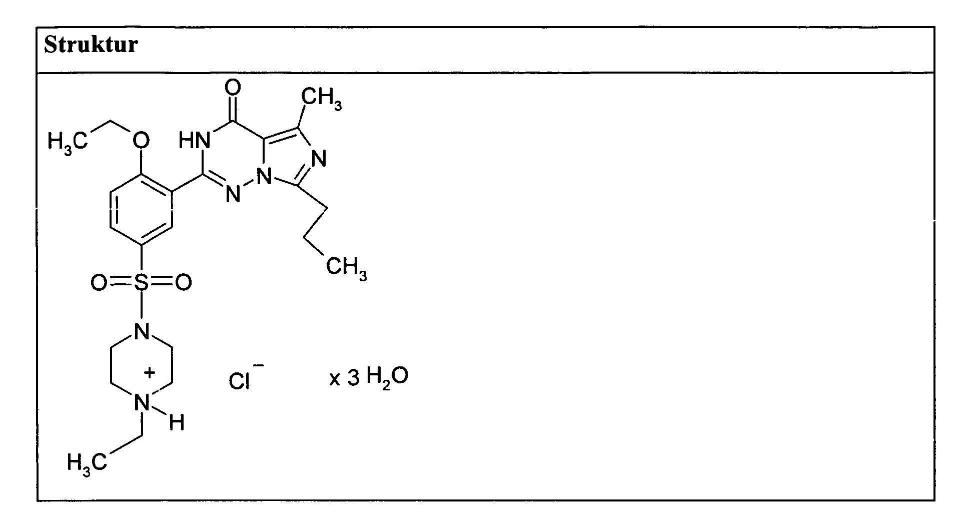

zur Herstellung von Arzneimitteln zur Behandlung von Herzinsuffizienz, Psoriasis, weiblicher Infertilität, Krebs, Diabetes, Augenerkrankungen wie Glaukom, Störungen der Magenbeweglichkeit, zystischer Fibrose, vorzeitigen Wehen, pulmonarem Bluthochdruck, Blasenerkrankungen, Prostatahyperplasie, nitrat-induzierte Toleranz, Präeklampsie, Alopecia, der Parkinson-Krankheit, Schmerz, Tinnitus oder dem renalen Syndrom.

Im Rahmen der Erfindung sind physiologisch unbedenkliche Salze bevorzugt. Physiologisch unbedenkliche Salze können Salze der erfindungsgemäßen Verbindungen mit anorganischen oder organischen Säuren sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Physiologisch unbedenkliche Salze können ebenso Metall- oder Ammoniumsalze der erfindungsgemäßen Verbindungen sein. Besonders bevorzugt sind z.B. Natrium-, Kalium-, Magnesium- oder Calciumsalze, sowie Ammoniumsalze, die abgeleitet sind von Ammoniak oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen, insbesondere die Salze, können auch als Hydrate vorliegen. Im Rahmen der Erfindung werden unter Hydraten solche Verbindungen verstanden, die im Kristall Wasser enthalten. Solche Verbindungen können ein oder mehrere, typischerweise 1 bis 5, Äquivalente Wasser enthalten. Hydrate lassen sich beispielsweise herstellen, indem man die betreffende Verbindung aus Wasser oder einem wasserhaltigen Lösungsmittel kristallisiert.

Solvate der erfindungsgemäßen Verbindungen sind stöchiometrische Zusammensetzungen der Verbindungen oder seinen Salzen mit Lösungsmittel.

Ein Acylrest mit 1 bis 3 Kohlenstoffatomen steht im Rahmen der Erfindung beispielsweise für Formyl, Acetyl oder Ethylcarbonyl.

Ein geradkettiger oder verzweigterAlkoxyrest mit 1 bis 3 Kohlenstoffatomen steht im Rahmen der Erfindung für Methoxy, Ethoxy, n-Propoxy, oder Isopropoxy.

Ein Alkoxycarbonylrest mit 1 bis 3 Kohlenstoffatomen steht im Rahmen der Erfindung für Methoxycarbonyl oder Ethoxycarbonyl.

Ein geradkettiger oder verzweigter Alkylrest mit 1 bis 5 oder 1 bis 3 Kohlenstoffatomen steht im Rahmen der Erfindung beispielsweise für Methyl, Ethyl, n-Propyl, Isopropyl, tert.Butyl, n-Pentyl. Bevorzugt sind geradkettige oder verzweigte Alkylreste mit 1 bis 4 bzw. 1 bis 3 Kohlenstoffatomen.

Halogen steht im Rahmen der Erfindung im allgemeinen für Fluor, Chlor, Brom und Jod. Bevorzugt sind Fluor, Chlor und Brom. Besonders bevorzugt sind Fluor und Chlor.

Eine weitere Ausführungsform der Erfindung betrifft die erfindungsgemäße Verwendung von Verbindungen der allgemeinen Formel (I), in welcher die Reste R¹⁶ und -SO₂NR³R⁴ in para-Position zueinander am Phenylring stehen und R¹, R², R³, R⁴ und R¹⁶ die oben angegebene Bedeutung haben.

Eine weitere Ausführungsform der Erfindung betrifft die erfindungsgemäße Verwendung von Verbindungen der allgemeinen Formel (Ia), 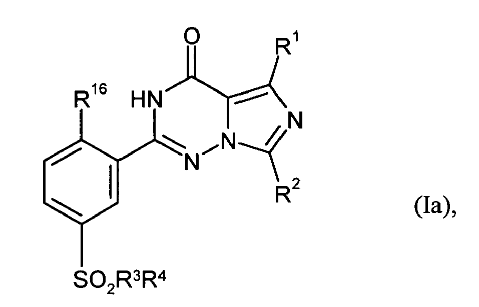 wobei R¹, R², R³, R⁴ und R¹⁶ die oben angegebene Bedeutung haben,
und deren Salze, Hydrate und/oder Solvate.

Bevorzugt ist die erfindungsgemäße Verwendung der folgenden Verbindungen:
2-[2-Ethoxy-5-(4-methyl-piperazin-1-sulfonyl)-phenyl]-5,7-dimethyl-3*H*-imidazo-[5,1-f]-[1,2,4]triazin-4-on;
2-[2-Ethoxy-5-(4-hydroxyethylpiperazine-1-sulfonyl)-phenyl]-5,7-dimethyl-3*H-*imidazo[5,1-f]-[1,2,4]triazin-4-on;
2-[2-Ethoxy-5-(4-hydroxypiperidine-1-sulfonyl)-phenyl]-5,7-dimethyl-3*H*-imidazo-[5,1-f]-[1,2,4]triazin-4-on;
2-[2-Ethoxy-5-(4-hydroxymethylpiperidin-1-sulfonyl)-phenyl]-5,7-dimethyl-3*H-*imidazo[5,1-f] [1,2,4]triazin-4-on;
2-[2-Ethoxy-5-(3-hydroxypyrrolidin-1-sulfonyl)-phenyl]-5,7-dimethyl-3*H*-imidazo-[5, 1-f]-[1,2,4]triazin-4-on;
4-Ethoxy-N-ethyl-N-(2-hydroxyethyl)-3-(5,7-dimethyl-4-oxo-3,4-dihydro-imidazo-[5,1-f]-[1,2,4]triazin-2-yl)benzolsulfonamid;
N,N-Diethyl-4-ethoxy-3-(5,7-dimethyl-4-oxo-3,4-dihydroimidazo[5,1-f][1,2,4]-triazin-2-yl)-benzolsulfonamid;
2-[2-Ethoxy-5-(4-(2-pyrimidinyl)-piperazin-1-sulfonyl)-phenyl]-5,7-dimethyl-3*H-*imidazo-[5,1-f][1,2,4]triazin-4-on;
2-[2-Ethoxy-5-(morpholin-4-sulfonyl)-phenyl]-5,7-dimethyl-3*H*-imidazo[5,1-f]-[1,2,4]triazin-4-on;
2-[2-Ethoxy-5-(1,4-dioxa-6-azaspiro[4.4]nonan-6-sulfonyl)-phenyl]-5,7-dimethyl-3*H*-imidazo[5,1-f][1,2,4]triazin-4-on;
N,N-Bis-(2-Methoxyethyl)-4-ethoxy-3-(5,7-dimethyl-4-oxo-3,4-dihydroimidazo[5,1-f]-[1,2,4]triazin-2-yl)-benzolsulfonamid;
N-(3-Isoxazolyl)-4-ethoxy-3-(5,7-dimethyl-4-oxo-3,4-dihydroimidazo[5,1-f][1,2,4]-triazin-2-yl)-benzolsulfonamid;
2-[2-Ethoxy-5-(2-t-butoxycarbonylaminomethylmorpholin-4-sulfonyl)-phenyl]-5,7-dimethyl-3*H*-imidazo[5,1-f][1,2,4]triazin-4-on;
2-[2-Ethoxy-5-(4-phenylpiperazin-1-sulfonyl)-phenyl]-5,7-dimethyl-3*H*-imidazo[5,1-f]-[1,2,4]triazin-4-on;
2-[2-Ethoxy-5-(3-hydroxy-3-methoxymethylpyrrolidin-1-sulfonyl)-phenyl]-5,7-dimethyl-3*H*-imidazo[5,1-f][1,2,4]triazin-4-on;
2-[2-Ethoxy-5-(4-methyl-piperazin-1-sulfonyl)-phenyl]-5-methyl-7-propyl-3*H-*imidazo [5,1-f][1,2,4]triazin-4-on;
2-[2-Ethoxy-5-(4-methyl-piperazin-1-sulfonyl)-phenyl]-5-methyl-7-propyl-3*H-*imidazo[5,1-f][1,2,4]triazin-4-on Lactat;
2-[2-Ethoxy-5-(4-methyl-piperazin-1-sulfonyl)-phenyl]-5-methyl-7-propyl-3*H-*imidazo[5,1-f][1,2,4]triazin-4-on Hydrochlorid;
2-[2-Ethoxy-5-(4-ethyl-piperazin-1-sulfonyl)-phenyl]-5-methyl-7-propyl-3*H-*imidazo[5,1-f][1,2,4]triazin-4-on;
2-[2-Ethoxy-5-(4-ethyl-piperazin-1-sulfonyl)-phenyl]-5-methyl-7-propyl-3*H-*imidazo[5,1-f][1,2,4]triazin-4-on Hydrochlorid;
2-[2-Ethoxy-5-(4-methyl-1-amino-piperazin-1-sulfonyl)-phenyl]-5-methyl-7-propyl-3*H*-imidazo[5,1-f][1,2,4]triazin-4-on;
2-[2-Ethoxy-5-(4-hydroxyethyl-1-amino-piperazin-1-sulfonyl)-phenyl]-5-methyl-7-propyl-3*H*-imidazo[5,1-f][1,2,4]triazin-4-on;
N,N-Bishydroxyethylaminoethyl-4-ethoxy-3-(5-methyl-4-oxo-7-propyl-3,4-dihydro-imidazo[5,1-f][1,2,4]triazin-2-yl)benzolsulfonamid;
2-[2-Ethoxy-5-(4-dimethoxyphosphorylmethyl-piperazin-1-sulfonyl)-phenyl]-5-methyl-7-propyl-3*H*-imidazo[5,1-f][1,2,4]triazin-4-on;
2-[2-Ethoxy-5-(4-diethoxyphosphorylmethyl-piperidin-1-sulfonyl)-phenyl]-5-methyl-7-propyl-3*H*-imidazo[5,1-f][1,2,4]triazin-4-on;
2-[2-Ethoxy-5-(4-hydroxy-piperidin-1-sulfonyl)-phenyl]-5-methyl-7-propyl-3*H-*imidazo[5,1-f][1,2,4]triazin-4-on;
2-{2-Ethoxy-5-[4-(2-hydroxy-ethyl)-piperazin-1-sulfonyl] -phenyl} -5-methyl-7-propyl-3*H*-imidazo[5,1-f][1,2,4]triazin-4-on;
2- {2-Ethoxy-5- [4-(2-hydroxy-ethyl)-piperazin-1-sulfonyl]-phenyl -5-methyl-7-propyl-3*H*-imidazo[5,1-f][1,2,4]triazin-4-on Hydrochlorid;
2-{2-Ethoxy-5-[4-(3-hydroxy-propyl)-piperazin-1-sulfonyl]-phenyl}-5-methyl-7-propyl-3*H*-imidazo[5,1-f][1,2,4]triazin-4-on;
N-Allyl-4-ethoxy-N-(2-hydroxy-ethyl)-3-(5-methyl-4-oxo-7-propyl-3,4-dihydro-imidazo[5,1-f][1,2,4]triazin-2-yl)benzolsulfonamid;
N-Ethyl-4-ethoxy-N-(2-hydroxy-ethyl)-3-(5-methyl-4-oxo-7-propyl-3,4-dihydro-imidazo[5,1-f][1,2,4]triazin-2-yl)benzolsulfonamid;
N,N-Diethyl-4-ethoxy-3-(5-methyl-4-oxo-7-propyl-3,4-dihydro-imidazo[5,1-f][1,2,4]triazin-2-yl)benzolsulfonamid;
N-(2-methoxyethyl)-3-(5-methyl-4-oxo-7-propyl-3,4-dihydro-imidazo[5,1-f][1,2,4]triazin-2-yl)-4-ethoxy-benzolsulfonsäureamid;
N-(2-N,N-dimethylethyl)-3-(5-methyl-4-oxo-7-propyl-3,4-dihydro-imidazo[5,1-f][1,2,4]triazin-2-yl)-4-ethoxy-benzolsulfonsäureamid;
N-[3-(1-morpholino)propyl]-3-(5-methyl-4-oxo-7-propyl-3,4-dihydro-imidazo[5,1-f] [1,2,4]triazin-2-yl)-4-ethoxy-benzolsulfonsäureamid;
N-{3-[1-(4-methyl)piperazino]-propyl} -3-(5-methyl-4-oxo-7-propyl-3,4-dihydro-imidazo[5,1-f][1,2,4]triazin-2-yl)-4-ethoxy-benzolsulfonsäureamid;
2-{2-Ethoxy-5-[4-(2-methoxy-ethyl)-piperazin-1-sulfonyl]-phonyl}-5-methyl-7-propyl-3*H*-imidazo[5,1-f][1,2,4]triazin-4-on;
2-{2-Ethoxy-5-[4-(2-N,N-dimethyl-ethyl)-piperazin-1-sulfonyl]-phenyl}-5-methyl-7-propyl-3*H*-imidazo[5,1-f][1,2,4]triazin-4-on;
2-{2-Ethoxy-5-[4-(3-N,N-dimethyl-propyl)-piperazin-1-sulfonyl]-phenyl}-5-methyl-7-propyl-3*H*-imidazo[5,1-f][1,2,4]triazin-4-on;
2-[2-Ethoxy-5-(4-dioxolano-piperidin-1-sulfonyl)-phenyl]-5-methyl-7-propyl-3*H-*imidazo[5,1-f][1,2,4]triazin-4-on;
2-[2-Ethoxy-5-(4-(S-methyl-4-furoxancarbonyl)-piperazin-1-sulfonyl)-phenyl]-5-methyl-7-propyl-3*H*-imidazo[5,1-f][1,2,4]triazin-4-on;
2-{2-Ethoxy-5-[4-acetyl-piperazin-1-sulfonyl]-phenyl}-5-methyl-7-propyl-3*H-*imidazo[5,1-f][1,2,4]triazin-4-on;
2-{2-Ethoxy-5-[4-formyl-piperazin-1-sulfonyl]-phenyl}-5-methyl-7-propyl-3*H-*imidazo[5,1-f][1,2,4]triazin-4-on;
2-[2-Ethoxy-5-(3-butylsydnonimin)-1-sulfonyl)-phenyl]-5-methyl-7-propyl-3*H-*imidazo[5,1-f][1,2,4]triazin-4-on;
5-Methyl-2-[5-(4-methyl-piperazin-1-sulfonyl)-2-propoxy-phenyl]-7-propyl-3*H-*imidazo[5,1-f][1,2,4]triazin-4-on;
5-Methyl-2-[5-(4-methyl-piperazin-1-sulfonyl)-2-propoxy-phenyl]-7-propyl-3*H-*imidazo[5,1-f][1,2,4]triazin-4-on Hydrochlorid;
2-[5-(4-Hydroxypiperidin-1-sulfonyl)-2-propoxy-phenyl]-5-methyl-7-propyl-3*H-*imidazo[5,1-f][1,2,4]triazin-4-on;
2-[5-(4-Hydroxymethylpiperidin-1-sulfonyl)-2-propoxy-phenyl]-5-methyl-7-propyl-3*H*-imidazo[5,1-f][1,2,4]triazin-4-on;
2-{5-[4-(2-hydroxyethyl)-piperazin-1-sulfonyl]-2-propoxy-phenyl}-5-methyl-7-propyl-3*H*-imidazo[5,1-f][1,2,4]triazin-4-on;
N-(1, 1-Dioxotetrahydro-1□⁶-thiophen-3-yl)-3-(5-methyl-4-oxo-7-propyl-3,4-dihydro-imidazo-[5,1-f][1,2,4]triazin-2-yl)-4-propoxy-benzolsulfonsäureamid;
N-(2-Dimethylaminoethyl)-N-methyl-3-(5-methyl-4-oxo-7-propyl-3,4-dihydro-imidazo[5,1-f][1,2,4]triazin-2-yl)-4-propoxy-benzolsulfonsäureamid;
3-(5-Methyl-4-oxo-7-propyl-3,4-dihydro-imidazo[5,1-f][1,2,4]triazin-2-yl)-N-(3-morpholin-4-yl-propyl)-4-propoxy-benzolsulfonsäureamid;
N,N-Bis-(2-hydroxyethyl)-3-(5-Methyl-4-oxo-7-propyl-3,4-dihydro-imidazo[5,1-f][1,2,4]triazin-2-yl)-4-propoxy-benzolsulfonsäureamid;
N-(3-Hydroxybenzyl)-3-(5-Methyl-4-oxo-7-propyl-3,4-dihydro-imidazo[5,1-f][1,2,4]triazin-2-yl)-4-propoxy-benzolsulfonsäureamid;
N-Ethyl-N-(2-hydroxyethyl)-3-(5-Methyl-4-oxo-7-propyl-3,4-dihydro-imidazo[5,1-f][1,2,4]triazin-2-yl)-4-propoxy-benzolsulfonsäureamid;
N-(3-Ethoxypropyl)-3-(5-Methyl-4-oxo-7-propyl-3,4-dihydro-imidazo[5,1-f]-[1,2,4]triazin-2-yl)-4-propoxy-benzolsulfonsäureamid;
2-[5(4-Hydroxypiperidin-1-sulfonyl)2-propoxy-phenyl]-5-methyl-7-propyl-3*H-*imidazo[5,1-*f*][1,2,4]triazin-4-on;
3-(5-Methyl-4-oxo-7-propyl-3,4-dihydro-imidazo[5,1-f][1,2,4]triazin-2-yl)-4-propoxy-N-pyridin-4-yl-benzolsulfonsäureamid;
N,N-Diethyl-3-(5-methyl-4-oxo-7-propyl-3,4-dihydro-imidazo[5,1-f][1,2,4]triazin-2-yl)-4-propoxy-benzolsulfonsäureamid;
1-[3-(5-methyl-4-oxo-7-propyl-3,4-dihydro-imidazo[5,1-f][1,2,4]triazin-2-yl)-4-propoxy-benzolsulfonyl]-piperidin-4-carbonsäure;
5-Methyl-2-[5-(morpholin-4-sulfonyl)-2-propoxy-phenyl]-7-propyl-3*H*-imidazo[5,1-*f*][1,2,4]triazin-4-on;
N-(2-Hydroxyethyl)-N-methyl-3-(5-methyl-4-oxo-7-propyl-3,4-dihydro-imidazo[5,1-f] [1,2,4]triazin-2-yl)-4-propoxy-benzolsulfonsäureamid;
N-(2-Hydroxyethyl)-3-(5-methyl-4-oxo-7-propyl-3,4-dihydro-imidazo[5,1-f][1,2,4]triazin-2-yl)-4-propoxy-N-propyl-benzolsulfonsäureamid;
N-[2-(3,4-Dimethoxy-phenyl)ethyl]-N-methyl-3-(5-methyl-4-oxo-7-propyl-3,4-dihydro-imidazo[5,1-f][1,2,4]triazin-2-yl)-4-propoxy-benzolsulfonsäureamid;
N-Allyl-N-(2-hydroxyethyl)-3-(5-methyl-4-oxo-7-propyl-3,4-dihydroimidazo[5,1-f][1,2,4]triazin-2-yl)-4-propoxybenzolsulfonsäureamid;
N-Allyl-N-cyclopentyl-3-(5-methyl-4-oxo-7-propyl-3,4-dihydroimidazo[5,1-f][1,2,4]triazin-2-yl)-4-propoxybenzolsulfonsäureamid;
N-Allyl-N-ethyl-3-(5-methyl-4-oxo-7-propyl-3,4-dihydroimidazo[5,1-f][1,2,4]triazin-2-yl)-4-propoxybenzolsulfonsäureamid;
2-[2-Ethoxy-4-methoxy-5-(4-methylpiperazin-1-sulfonyl)-phenyl]-5-methyl-7-propyl-3H-imidazo[5, 1-f][1,2,4]triazin-4-on;
2- {2-Ethoxy-5-[4-(2-hydroxyethyl)-piperazin-1-sulfonyl]-4-methoxy-phenyl}-5 - methyl-7-propyl-3H-imidazo[5,1-f][1,2,4]triazin-4-on;
4-Ethoxy-N-ethyl-N-(2-hydroxyethyl)-2-methoxy-5-(5-methyl-4-oxo-7-propyl-3,4-dihydro-imidazo[5,1-f][1,2,4]triazin-2-yl)-benzolsulfonsäureamid;
4-Ethoxy-N-(4-ethoxyphenyl)-2-methoxy-5-(5-methyl-4-oxo-7-propyl-3,4-dihydro-imidazo[5,1-f][1,2,4]triazin-2-yl)-benzolsulfonsäureamid;
4-Ethoxy-N-ethyl-N-(2-hydroxy-ethyl)-3-(5-ethyl-4-oxo-7-propyl-3,4-dihydroimidazo [5,1-f][1,2,4]triazin-2-yl)benzolsulfonsäureamid;
N-(2-methoxyethyl)-3-(5-ethyl-4-oxo-7-propyl-3,4-dihydro-imidazo[5,1-f][1,2,4]triazin-2-yl)-4-ethoxybenzolsulfonsäureamid;
N,N-Bis-(2-Methoxyethyl)-3-(5-ethyl-4-oxo-7-propyl-3,4-dihydroimidazo[5,1-f][1,2,4]tri-azin-2-yl)-4-ethoxybenzolsulfonsäureamid;
2-[5-(4-Hydroxypiperidin-1-sulfonyl)-2-ethoxyphenyl]-5-ethyl-7-propyl-3*H-*imidazo[5,1-*f*]-[1,2,4]triazin-4-on;
2-[5-(4-Hydroxymethylpiperidin-1-sulfonyl)-2-ethoxy-phenyl]-5-ethyl-7-propyl-3*H-*imidazo[5,1-f](1,2,4]triazin-4-on;
2-{2-Ethoxy-5-[4-(2-hydroxyethyl)-piperazin-1-sulfonyl]-phenyl}-5-ethyl-7-propyl-3*H*-imidazo[5,1-f][1,2,4]triazin-4-on;
2-[2-Ethoxy-5-(4-methylpiperazin-1-sulfonyl)-phenyl]-5-ethyl-7-propyl-3*H-*imidazo[5,1-f]-[1,2,4]triazin-4-on;
2-[2-Ethoxy-5-(4-methylpiperazin-1-sulfonyl)-phenyl]-5-ethyl-7-propyl-3*H-*imidazo[5,1-f][1,2,4]triazin-4-on Hydrochlorid;
3-(5-Ethyl-4-oxo-7-propyl-3,4-dihydroimidazo[5,1-f][1,2,4]triazin-2-yl)-N-(3-morpholin-4-yl-propyl)-4-ethoxybenzolsulfonsäureamid;
N-(2-Hydroxyethyl)-3-(5-ethyl-4-oxo-7-propyl-3,4-dihydro-imidazo[5,1-f][1,2,4]triazin-2-yl)-4-ethoxy-N-propyl-benzolsulfonsäureamid;
2-[2-Ethoxy-5-(4-ethyl-piperazin-1-sulfonyl)-phenyl]-5-methyl-7-propyl-3H-imidazol[5,1 -f][1,2,4]triazin-4-on Hydrochlorid-Trihydrat;
2-[2-Ethoxy-5-(4-ethyl-piperazin-1-sulfonyl)-phenyl]-5-methyl-7-propyl-3H-imidazo[5,1-f] [1,2,4]triazin-4-on Dihydrochlorid.

Die erfindungsgemäßen Verbindungen können gemäß der Beschreibung in der WO 99/24433 hergestellt werden, auf deren diesbezügliche Offenbarung hier ausdrücklich Bezug genommen wird.

Die erfindungsgemäßen Verbindungen die c-GMP metabolisierende Phosphodiesterase 5. Dies führt zu einem Anstieg von c-GMP. Die differenzierte Expression der Phosphodiesterasen in verschiedenen Zellen, Geweben und Organen, ebenso wie die differenzierte subzelluläre Lokalisation dieser Enzyme, ermöglichen in Verbindung mit den erfindungsgemäßen selektiven Inhibitoren, eine selektive Adressierung der verschiedenen von cGMP regulierten Vorgänge.

Außerdem verstärken die erfindungsgemäßen Verbindungen die Wirkung von Substanzen, wie beispielsweise EDRF (Endothelium derived relaxing factor), ANP (atrial natriuretic peptide), von Nitrovasodilatoren und allen anderen Substanzen, die auf eine andere Art als Phosphodiesterase-Inhibitoren die cGMP-Konzentration erhöhen.

Daher sind die erfindungsgemäßen Verbindungen der allgemeine Formel (I) geeignet zur Prophylaxe und/oder Behandlung von Erkrankungen, bei denen ein Anstieg der cGMP-Konzentration heilsam ist, d.h. Erkrankungen, die im Zusammenhang mit cGMP-regulierten Vorgängen stehen (im Englischen meist einfach als 'cGMP-related diseases' bezeichnet). Gemäß der vorliegenden Erfindung handelt es sich hierbei um Herzinsuffizienz, Psoriasis, weiblicher Infertilität, Krebs, Diabetes, Augenerkrankungen wie Glaukom, Störungen der Magenbeweglichkeit, zystischer Fibrose, vorzeitigen Wehen, pulmonarem Bluthochdruck, Blasenerkrankungen, Prostatahyperplasie, nitrat-induzierte Toleranz, Präeklampsie, Alopecia, der Parkinson-Krankheit, Schmerz, Tinnitus oder dem renalen Syndrom.

Eine zeitweilige oder dauerhafte Schädigung der Augen kann aufgrund einer Blutgefäßverengung und einer daraus folgenden mangelhaften Versorgung des Auges mit Nährstoffen entstehen. Beispielsweise kann dies - neben einem zu hohen Intraoculardruck - eine der Ursachen für ein Glaukom sein (vgl. z.B. Van de Voorde, J. Invest. Ophthal. & Vis. Sci. 39(9):1642-1646 (1998)). Es gibt Berichte über eine Verlangsamung des Fortschreitens glaukomatöser optischer Neuropathie bei systemischer Gabe eines NO-Donors, was auf eine Erweiterung der Blutgefäße in den Augen zurückzuführen sein könnte (vgl. Afshari, Invest. Ophthalmol. Vis. Sci. 38(Suppl.):S277 (1997); Grunwald, British J. Ophthal. 83(2):162-167 (1999)). Inhibitoren der cGMP PDE führen ― wie vorstehend beschrieben ― analog zu NO-Donoren zu einer Erhöhung des cGMP-Spiegels und können damit unter anderem eine Vasodilatation der Blutgefäße in den Augen hervorrufen und somit zur Behandlung von Glaukomen verwendet werden.

Grundsätzlich können die Verbindungen der Formel (I) jedoch auch zur Behandlung anderer Erkankungen des Auges verwendet werden, beispielsweise zur Behandlung oder Prophylaxe von zentraler retinaler oder posteriorer cilliarer Arterienokklusion, zentraler retinaler Venenokklusion, optischer Neuropathie wie anteriorer ischämischer optischer Neuropathie und glaukomatöser optischer Neuropathie, sowie von makulärer Degeneration.

Die Aktivität der Verbindungen der Formel (I) als Inhibitoren der Phosphordiesterasen (PDEs) ist in der WO 99/24433 beschrieben, auf deren diesbezüglichen Inhalt hier ausdrücklich Bezug genommen wird.

Die Wirkstoffe sowie ihre physiologisch unbedenklichen Salze (z.Bsp. Hydrochloride, Maleinate oder Lactate) können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 9 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Die Applikation erfolgt in üblicher Weise, vorzugsweise oral, transdermal oder parenteral, z.Bsp. perlingual , buccal, intravenös, nasal, rektal oder inhalativ.

Für die Anwendung beim Menschen werden bei oraler Administration Dosierungen von 0,001 bis 50 mg/kg vorzugsweise 0,01 mg/kg - 20 mg/kg sinnvollerweise verabreicht. Bei parenteraler Administration, wie z.B. über Schleimhäute nasal, buccal, inhalativ, ist eine Dosierung von 0,001 mg/kg - 0,5 mg/kg sinnvoll.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchen die Verabreichung erfolgt. So kann es in einigen Fällen ausreichend sein, mit weniger als der oben genannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die erfindungsgemäßen Verbindungen sind auch zur Anwendung in der Tiermedizin geeignet. Für Anwendungen in der Tiermedizin können die Verbindungen oder ihre nicht toxischen Salze in einer geeigneten Formulierung in Übereinstimmung mit den allgemeinen tiermedizinischen Praxen verabreicht werden. Der Tierarzt kann die Art der Anwendung und die Dosierung nach Art des zu behandelnden Tieres festlegen.

## Patentansprüche

1. Verwendung von 2-Phenyl-substituierten Imidazotriazinonen der allgemeinen Formel (I) 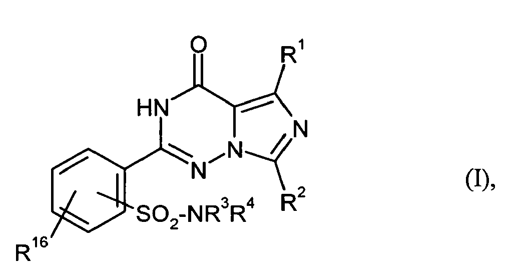 in welcher
R¹ für Methyl oder Ethyl steht,
R² für Ethyl oder Propyl steht,
R³ und R⁴ gleich oder verschieden sind und für eine geradkettige oder verzweigte Alkylkette mit bis zu 5 Kohlenstoffatomen stehen, die gegebenenfalls bis zu zweifach gleich oder verschieden durch Hydroxy oder Methoxy substituiert ist,
oder
R³ und R⁴ gemeinsam mit dem Stickstoffatom einen Piperidinyl-, Morpholinyl-, Thiomorpholinylring oder einen Rest der Formel 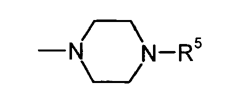 bilden,
worin
R⁵ Wasserstoff, Formyl, Acyl oder Alkoxycarbonyl mit jeweils bis zu 3 Kohlenstoffatomen bedeutet,
oder geradkettiges oder verzweigtes Alkyl mit bis zu 3 Kohlenstoffatomen bedeutet, das gegebenenfalls ein- bis zweifach, gleich oder verschieden durch Hydroxy, Carboxyl, geradkettiges oder verzweigtes Alkoxy oder Alkoxycarbonyl mit jeweils bis zu 3 Kohlenstoffatomen oder durch Gruppen der Formeln -(D)ₐ₋NR⁶R⁷ oder -P(O)(OR⁸)(OR⁹) substituiert ist,
worin
a eine Zahl 0 oder 1 bedeutet,
D eine Gruppe der Formel -CO bedeutet,
R⁶ und R⁷ gleich oder verschieden sind und Wasserstoff oder Methyl bedeuten,
R⁸ und R⁹ gleich oder verschieden sind und Wasserstoff, Methyl oder Ethyl bedeuten,
oder
R⁵ Cyclopentyl bedeutet,
und die unter R³ und R⁴ aufgeführten, gemeinsam mit dem Stickstoffatom gebildeten Heterocyclen, gegebenenfalls ein- bis zweifach, gleich oder verschieden, gegebenenfalls auch geminal, durch Hydroxy, Formyl, Carboxyl, Acyl oder Alkoxycarbonyl mit bis jeweils zu 3 Kohlenstoffatomen oder Gruppen der Formeln -P(O)(OR¹⁰)(OR¹¹) oder -(CO)_{b}NR¹²R¹³ substituiert sind,
worin
R¹⁰ und R¹¹ gleich oder verschieden sind und Wasserstoff, Methyl oder Ethyl bedeuten,
b eine Zahl 0 oder 1 bedeutet,
und
R¹² und R¹³ gleich oder verschieden sind und Wasserstoff oder Methyl bedeuten
und/oder die unter R³ und R⁴ aufgeführten, gemeinsam mit dem Stickstoffatom gebildeten Heterocyclen, gegebenenfalls durch geradkettiges oder verzweigtes Alkyl mit bis zu 3 Kohlenstoffatomen substituiert sind, das gegebenenfalls ein- bis zweifach, gleich oder verschieden durch Hydroxy, Carboxyl oder durch einen Rest der Formel P(O)OR¹⁴OR¹⁵ substituiert ist,
worin
R¹⁴ und R¹⁵ gleich oder verschieden sind und Wasserstoff, Methyl oder Ethyl bedeuten,
und/oder die unter R³ und R⁴ aufgeführten, gemeinsam mit dem Stickstoffatom gebildeten Heterocyclen, gegebenenfalls durch über N-verknüpftes Piperidinyl oder Pyrrolidinyl substituiert sind,
und
R¹⁶ für Ethoxy oder Propoxy steht,
und deren Salze, Hydrate und/oder Solvate, zur Herstellung von Arzneimitteln zur Behandlung von Blasenerkrankungen, Prostatahyperplasie, Tinnitus, zystischer Fibrose, Psoriasis, weiblicher Infertilität, Krebs, Diabetes, Augenerkrankungen wie Glaukom, Störungen der Magenbeweglichkeit, vorzeitigen Wehen, , nitrat-induzierte Toleranz, Präeklampsie, Alopecia, der Parkinson-Krankheit, Schmerz, oder dem renalen Syndrom.

2. Verwendung nach Anspruch 1 von Verbindungen der allgemeinen Formel (Ia), 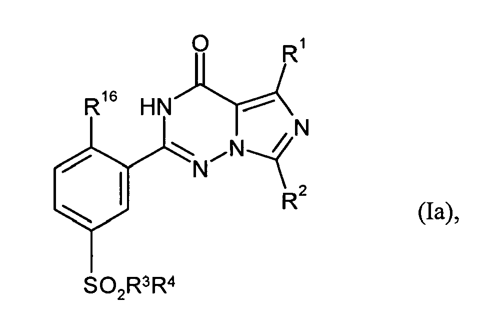 wobei R¹, R², R³, R⁴ und R¹⁶ die im Anspruch 1 angegebenene Bedeutung haben,
und deren Salze, Hydrate und/oder Solvate.

3. Verwendung von 2-Phenyl-substituierten Imidazotriazinonen gemäß Anspruch 1 oder 2 mit folgenden Strukturen: 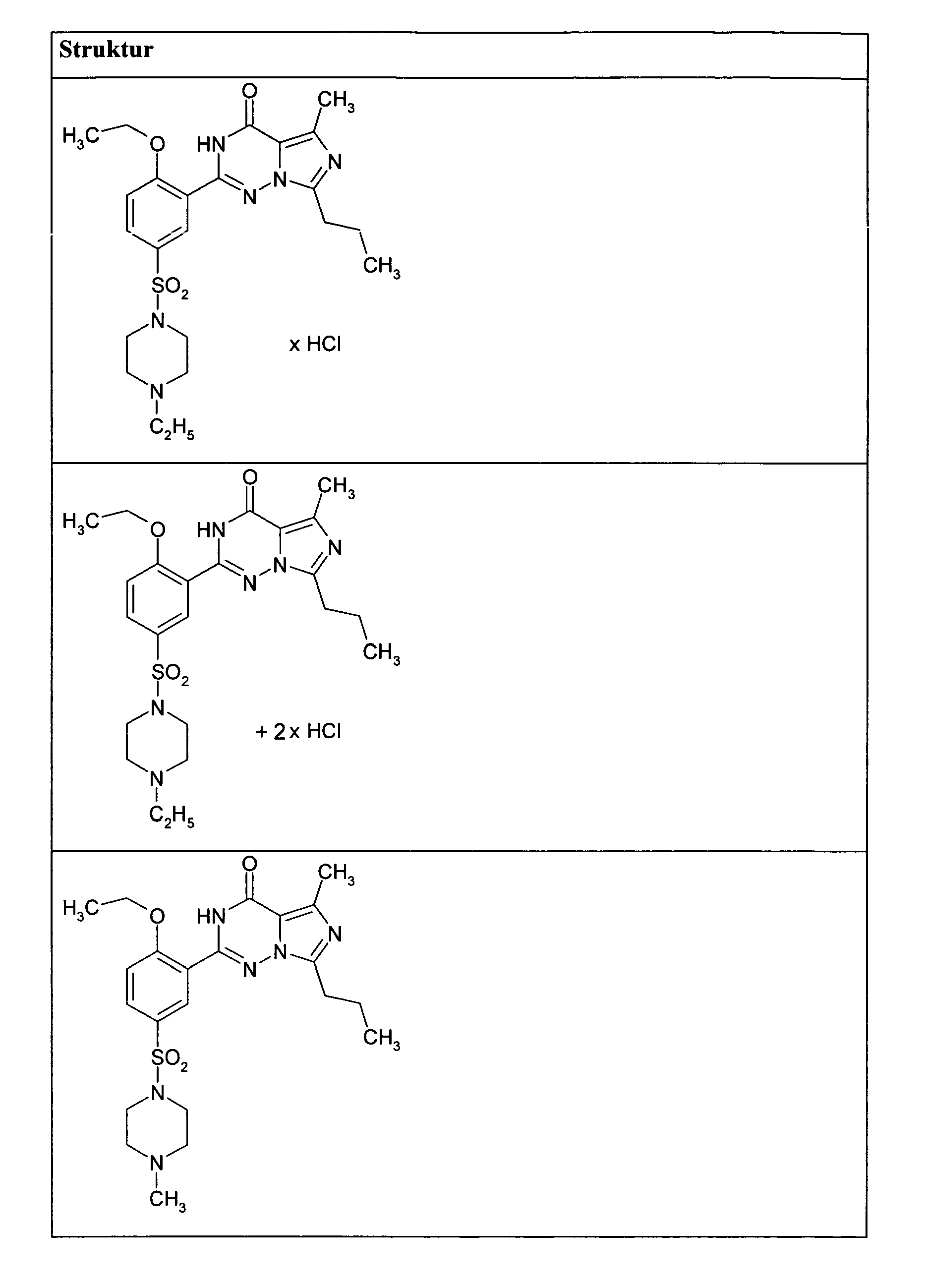 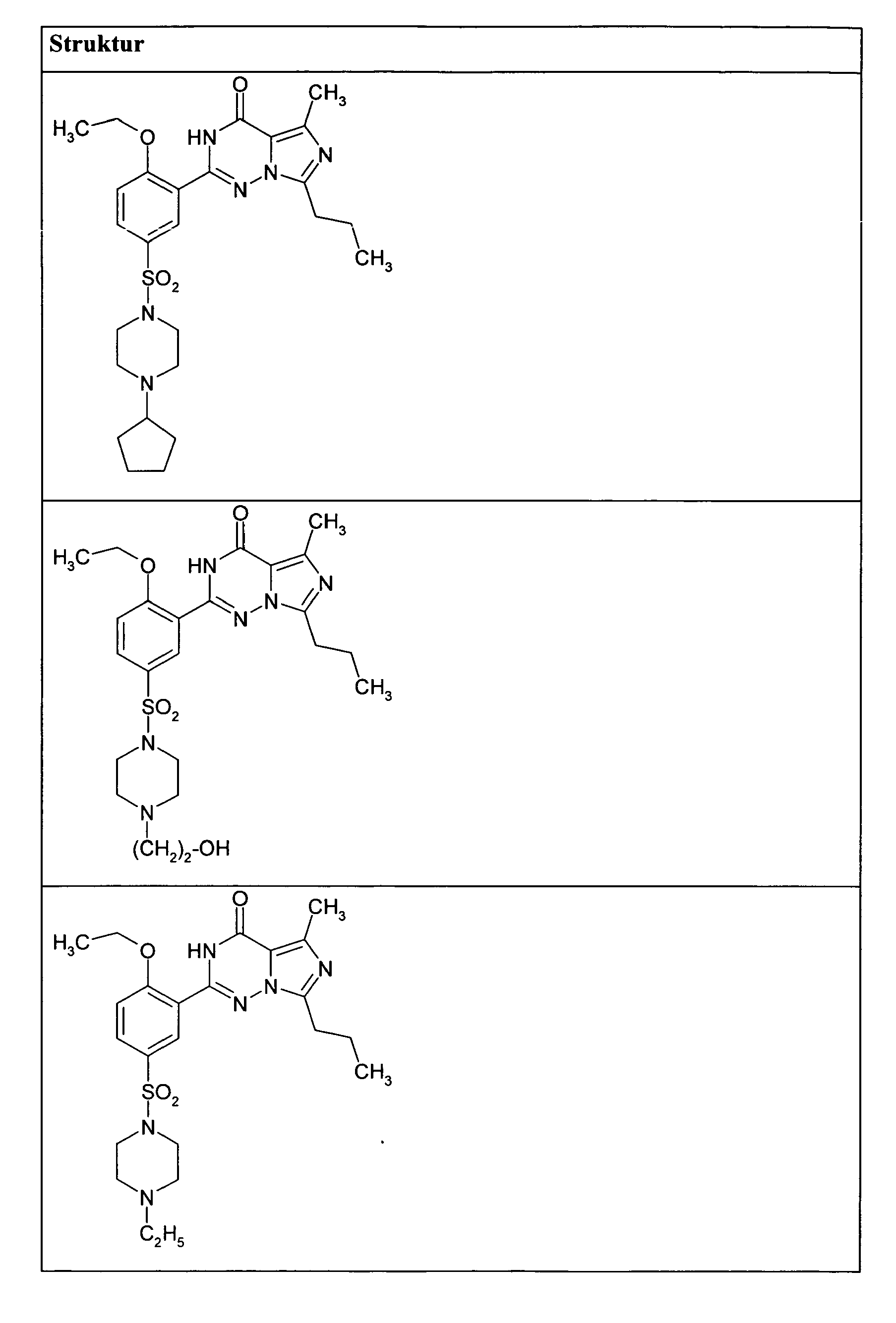 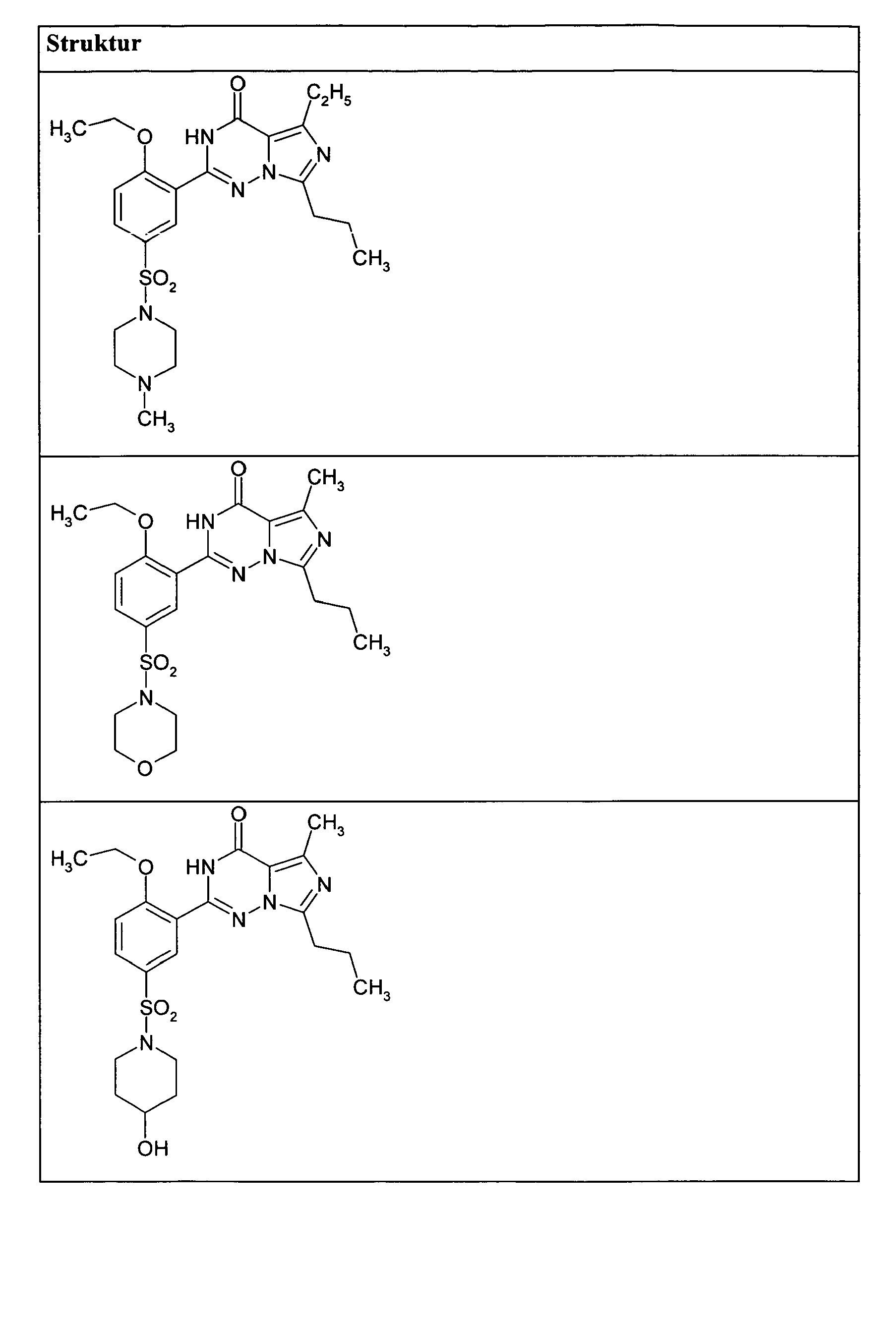 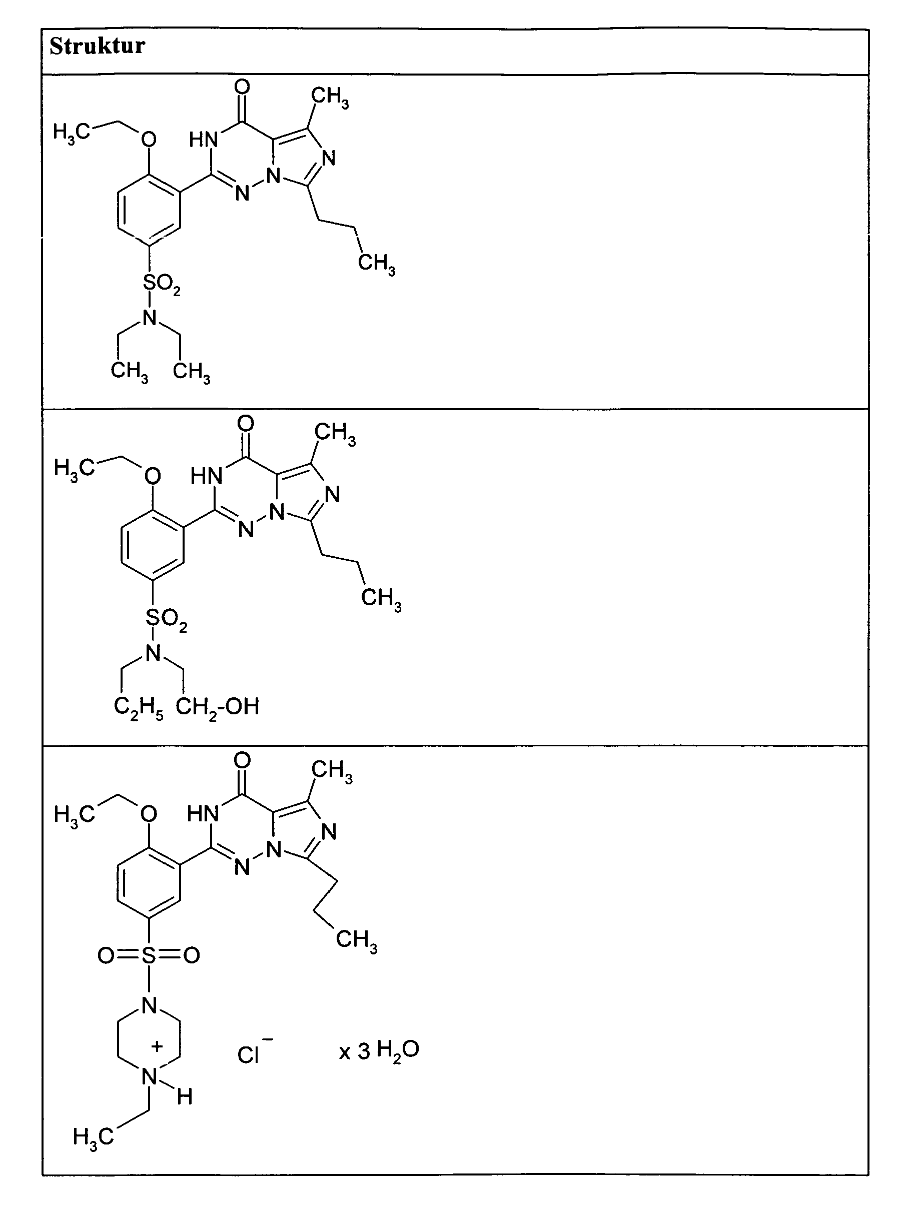 zur Herstellung von Arzneimitteln zur Behandlung von Blasenerkrankungen, Prostatahyperplasie, Tinnitus, zystischer Fibrose, Psoriasis, weiblicher Infertilität, Krebs, Diabetes, Augenerkrankungen wie Glaukom, Störungen der Magenbeweglichkeit, vorzeitigen Wehen, nitrat-induzierte Toleranz, Präeklampsie, Alopecia, der Parkinson-Krankheit, Schmerz oder dem renalen Syndrom.
